(19) Europäisches Patentamt
European Patent Office
Office européen des brevets

(11) Veröffentlichungsnummer: **0 327 490**
**A1**

(12) # EUROPÄISCHE PATENTANMELDUNG

(21) Anmeldenummer: **89730021.6**

(22) Anmeldetag: **01.02.89**

(51) Int. Cl.⁴: **A 61 K 49/00**

(30) Priorität: **05.02.88 DE 3803972**
**05.02.88 DE 3803971**

(43) Veröffentlichungstag der Anmeldung:
**09.08.89** Patentblatt **89/32**

(84) Benannte Vertragsstaaten: **ES GR**

(71) Anmelder: **SCHERING AKTIENGESELLSCHAFT Berlin
und Bergkamen
Müllerstrasse 170/178 Postfach 65 03 11
D-1000 Berlin 65 (DE)**

(72) Erfinder: **Stein, Michael, Dr.
Holsteinische Strasse 42
D-1000 Berlin 31 (DE)**

**Heldmann, Dieter
Lindauer Allee 85
D-1000 Berlin 51 (DE)**

**Fritzsch, Thomas, Dr.
Elisenstrasse 2
D-1000 Berlin 41 (DE)**

**Siegert, Joachim, Dr.
Weddingstrasse 5
D-1000 Berlin 65 (DE)**

**Rössling, Georg, Dr.
Oranienburger Chaussee 60c
D-1000 Berlin 28 (DE)**

**Speck, Ulrich, Prof.
Benediktiner Strasse 50
D-1000 Berlin 28 (DE)**

(74) Vertreter: **Maikowski, Michael, Dipl.-Ing. Dr.
Xantener Strasse 10
D-1000 Berlin 15 (DE)**

(54) **Ultraschallkontrastmittel, Verfahren zu deren Herstellung und deren Verwendung als Diagnostika und Therapeutika.**

(57) Die Erfindung betrifft Ultraschallkontrastmittel bestehend aus Mikropartikeln, die aus Amylosen oder synthetischen, bioabbaubaren Polymeren und einem Gas und/oder einer Flüssigkeit mit einem Siedepunkt unter 60°C bestehen, Verfahren zu deren Herstellung und deren Verwendung als Diagnostika und Therapeutika

EP 0 327 490 A1

## Beschreibung

### Ultraschallkontrastmittel, Verfahren zu deren Herstellung und deren Verwendung als Diagnostika und Therapeutika

Die Erfindung betrifft Mikropartikel nach dem Oberbegriff des Patentanspruchs 1, Verfahren zu deren Herstellung und deren Verwendung als Diagnostika und Therapeutika.

Es ist bekannt, daß durch periphere Injektion von Lösungen, die feine Gasblasen enthalten, cardiale Echokontraste erzielt werden können (Roelandt J, Ultrasound Med Biol 8:471-492, 1982). Diese Gasblasen werden in physiologisch verträglichen Lösungen z. B. durch Schütteln, andere Agitation oder durch Zusatz von Kohlendioxid erhalten. Sie sind jedoch hinsichtlich Anzahl und Größe nicht standardisiert und können nur unzulänglich reproduziert werden. Auch sind sie in der Regel nicht stabilisiert, so daß ihre Lebensdauer gering ist. Ihre mittleren Durchmesser liegen meist über Erythrocytengröße, so daß keine Lungenkapillarpassage mit nachfolgender Konstrastierung von Organen wie linkes Herz, Leber, Niere oder Milz möglich ist. Darüberhinaus eignen sie sich nicht für Quantifizierungen, da sich das von ihnen erzeugte Ultraschallecho aus mehreren, nicht voneinander zu trennenden Prozessen wie Blasenentstehung, Koaleszenz und Auflösung zusammensetzt. So ist es z. B. nicht möglich, mit Hilfe dieser Ultraschall-Kontrastmittel über die Messung des Kontrastverlaufs im Myokard Aussagen über die Transitzeiten zu gewinnen. Hierzu sind Kontrastmittel notwendig, deren Streukörper keiner eigenen Kinetik unterliegen.

Daneben gibt es Ultraschallkontrastmittel in Form von Partikeln (Ophir, Gobuty, Mc Whirt, Maklad, Ultrasonic Backscatter from Contrast-producing Collagen Microspheres, Ultrasonic Imaging 2:66-67, 1980). Ferner werden (Ophir, Mc Whirt, Maklad, Aqueous Solutions as Potential Ultrasonic Contrast Agents, Ultrasonic Imaging 1:265-279, 1979 sowie Tyler, Ophir, Maklad, In-vivo Enhancement of Ultrasonic Image Luminance by Aqueous Solutions with High Speed of Sound, Ultrasonic Imaging 3:323-329, 1981) Lösungen höherer Dichte als Ultra schall-Kontrastmittel eingesetzt. Es ist auch bekannt, Emulsionen als Ultraschall-Kontrastmittel zu verwenden (Mattrey, Andre, Ultrasonic Enhancement of Myocardial Infarction with Perfluorcarbon Compounds in Dogs, Am J Cardiol 54:206-210, 1984).

Es hat sich gezeigt, daß die gasfreien Kontrastmittel insgesamt nur eine geringe Effizienz besitzen. Die gashaltigen Zubereitungen haben den Nachteil einer nur geringen in-vivo Stabilität. Darüberhinaus ist die Größe der Gasblasen meistens nicht standardisierbar. Ausreichende Kontrasteffekte sind im arteriellen Gefäßsystem nach peripher venöser Injektion in aller Regel nicht möglich.

In den EP A2 123 235 und 0 122 624 werden Gasbläschen enthaltende Ultraschall-Kontrastmittel beschrieben, die die Lungenkapillaren passieren können und damit den gewünschten Kontrasteffekt bewirken.

Die EP A2 0 224 934 beschreibt Ultraschall-Kontrastmittel in Form von gasgefüllten Gelatine- oder Albuminhohlkörpern. Nachteilig ist jedoch die Verwendung von körperfremden oder denaturierten körpereigenen Eiweißen wegen des damit verbundenen allergenen Risikos.

Mit keinem der bisher bekannten Ultraschallkontrastmittel gelingt eine Organdarstellung mit ausreichender Signalintensität durch selektive Anreicherung nach i.v. Gabe. Quantifizierungen sind daher z.Z. nicht möglich.

Der Erfindung liegt die Aufgabe zugrunde, Ultraschallkontrastmittel auf der Basis von Mikropartikeln herzustellen, die neben bestimm- und reproduzierbaren Volumina eine erheblich längere Lebensdauer, als bisher bekannt, aufweisen, gute Verträglichkeit ohne allergenes Potential besitzen und intrazellulär in RES und damit auch in der Leber oder Milz angereichert werden können.

Erfindungsgemäß wird diese Aufgabe durch Mikropartikel, die aus Amylosen oder einem synthetischen, bioabbaubaren Polymeren und einem Gas und/oder einer Flüssigkeit mit einem Siedepunkt unter 60° C bestehen, gelöst.

Als synthetische, bioabbaubare Polymere sind Polyester von $\alpha$-, $\beta$-, $\gamma$- oder $\varepsilon$-Hydroxycarbonsäuren, Polyalkylcyanoacrylate, Polyaminosäuren, Polyamide, polyacrylierte Saccharide oder Polyorthoester zu nennen.

Als besonders geeignet haben sich
Polymilchsäure,
Poly-$\varepsilon$-caprolacton,
Copolymeres aus Milchsäure und Glykolsäure oder $\varepsilon$-caprolacton,
Polyhydroxybuttersäure,
Polyhydroxyvaleriansäure
Copolymeres aus Hydroxybutter- und Hydroxyvaleriansäure
Polymere aus Glutaminsäure und/oder Lysin,
Polydioxanon
Polymeres oder Copolymeres aus Aminosäuren oder/und Terephthalsäure, Phthalsäure oder Sebacylsäure,
Polyacryldextran,
Polyacrylstärke,
Polyacrylamid,
Polyurethan,
Polyester,
Polyacetal,
Polyaminotriazol oder
Polyalkylcyanoacrylate
erwiesen.

Stärke oder Stärkederivate können ebenso in den Mikropartikeln enthalten sein. Als geeignet haben sich Amylosen erwiesen, da sich diese Stärkederivate durch gute Wasserlöslichkeit und der Fähigkeit zur Bildung von Einschlußverbindungen auszeichnen.

Als besonders geeignete Amylosen sind die Cyclodextrine und deren Derivate, beispielsweise $\alpha$-, $\beta$-, und $\gamma$-Cyclodextrin zu nennen.

Die Mikropartikel enthalten Gase und/oder Flüssigkeiten mit einem Siedepunkt unter 60° C in freier oder gebundener Form. Die Verwendung eines Gas-Flüssigkeits-Gemisches in den Ultraschall-Kontrastmitteln ist ebenfalls möglich.

Als Gase können beispielsweise Luft, Stickstoff, Edelgase, Wasserstoff, Kohlendioxid, Ammoniak, Sauerstoff, Methan, Ethan, Propan, Butan, Ethylen oder andere Kohlenwasserstoffe oder deren Gemische verwendet werden.

Als einschließbare Flüssigkeiten werden bevorzugt
1.1-Dichlorethylen,
2-Methyl-2-buten,
Isopropylchlorid,
2-Methyl-1.3-butadien,
2-Butin,
2-Methyl-1-buten,
Dibromdifluormethan,
Furan,
3-Methyl-1-buten,
Isopentan,
Diethylether,
3.3-Dimethyl-1-butin,
Dimethylaminoaceton,
Propylenoxid,
N-Ethylmethylamin,
Brommethan,
N-Ethyldimethylamin,
Methylenchlorid,
Pentan,
Cyclopentan,
2,3-Pentadien,
Cyclopenten
oder deren Gemische verwendet.

Mit Vorteil können die Mikropartikel auch Substanzen mit niedrigen Dampfdrücken und/oder niedrigen Siedepunkten, insbesondere ätherische Öle enthalten.

Besonders vorteilhaft ist es, die aus Amylosen bestehenden Mikropartikel mit einer Hüllsubstanz zu überziehen. Dabei können die Mikropartikel von Ölen, Fetten und/oder oberflächenaktiven Substanzen umhüllt und in wäßrigem Medium suspendiert sein.

Besonders vorteilhaft ist es, die aus Amylosen bestehenden Mikropartikel mit einer Matrix, insbesondere polymerer Struktur zu umhüllen.

Durch Zugabe osmotisch aktiver Substanzen, beispielsweise Kochsalz, Galaktose, Glukose, Fruktose kann die physiologische Isotonie eingestellt werden.

Die Erfindung betrifft auch Verfahren zur Herstellung von Ultraschallkontrastmitteln, die aus synthetischen, bioabbaubaren Polymeren bestehen.

Ein vorteilhaftes Verfahren zur Herstellung der erfindungsgemäßen Ultraschallkontrastmittel besteht darin, daß ein Polymer oder ein Copolymer in einem oder mehreren mit Wasser nicht mischbaren, organischen Lösungsmitteln gelöst und anschließend ggf. nach Zusatz eines weiteren Lösungsmittels in Wasser emulgiert wird und die erhaltende Emulsion anschließend filtriert, ggf. getrocknet wird.

Ein weiteres Verfahren besteht darin, daß ein Polymer oder ein Copolymer in einem oder mehreren Gasblasen enthaltenden Lösungsmitteln gelöst und anschließend ggf. nach Zusatz eines weiteren Lösungsmittels oder eines weiteren Polymeren ausfällt oder in Wasser emulgiert werden und die erhaltende Suspension oder Emulsion anschließend filtriert, ggf. getrocknet wird. Zur Aufarbeitung ist auch das Gefriertrocknungsverfahren geeignet.

Mit Vorteil können die erhaltenen Produkte fein gemahlen werden.

Bei den beschriebenen Verfahren werden als Lösungsmittel beispielsweise Furan, Pentan, Aceton, Dioxan, Ethylacetat, Xylol, Methylenchlorid, Cyclohexan oder Hexan oder Lösungsmittelgemische verwendet. Der Emulsion können auch Emulgatoren zugesetzt werden.

In einer anderen Variante des Herstellungsverfahrens wird nicht von einem Polymeren ausgegangen, sondern von Monomeren, aus denen das Polymer gebildet wird. Dabei wird so gearbeitet, daß ein Monomer in einem oder mehreren organischen Lösungsmitteln gelöst und in 5 -30 Teilen Wasser oder 0,01 - 0,1 N Salzsäure ggf. unter Zusatz von Emulgatoren oder Puffersubstanzen bei einer Temperatur unterhalb des Siedepunkts des organischen Lösungsmittels emulgiert wird und dieser Emulsion eine 0,2% - 20%ige wäßrige Lösung eines zweiten Monomeren oder ggf. die Lösung einer pH-Wert erhöhenden Substanz zugegeben und ggf. getrocknet wird.

Bei einer anderen Arbeitsweise wird ein Monomer in einer oder mehreren gasblasenenthaltenden Flüssigkeiten ggf. unter Zusatz von Emulgatoren oder Puffersubstanzen gelöst oder dispergiert. Zu dieser Lösung oder Dispersion wird ggf. eine 0,2% - 20%ige Lösung eines zweiten Monomeren oder eine pH-Wert erhöhende Substanz in gelöster oder gasförmiger Form gegeben und ggf. getrocknet.

Beispielsweise wird als erstes Monomer Terephthaloyl-oder Sebacoylchlorid oder Cyanacrylsäureester, als zweites Monomer L-Lysin und als organisches Lösungsmittel beispielsweise 2-Methyl-1.3-butadien, Dioxan, Methylenchlorid, Toluol oder Cyclohexan verwendet.

Gemäß einem weiteren Verfahren werden die Ultraschallkontrastmittel dadurch hergestellt, daß in einer 0,5 -10%igen wäßrigen Lösung oder Dispersion eines Monomeren, die ggf. Zusätze wie Emulgatoren (0,01 - 5 %) oder Quasiemulgatoren (0,1 - 5 %) enthält, Gasblasen erzeugt und danach eine quervernetzende Substanz und/oder ein Reaktionsstarter zugesetzt werden.

Die im vorstehend beschriebenen Ultraschallkontrastmittel können sowohl für diagnostische als auch für therapeutische Verfahren verwendet werden.

Die Applikation der Mittel erfolgt beispielsweise durch Injektionen.

Die Erfindung wird durch folgende Beispiele erläutert:

Beispiel 1:

500 mg Polylactid werden in 4 ml Furan und 0,6 ml Cyclohexan gelöst und diese Lösung in 40 ml einer 0,1%igen Lösung von Polyoxyethylenpolyoxypropylen-Polymer mit Molekulargewicht 12.000 (Pluronic® F 127) enthält, emulgiert, wobei die Temperatur während des Emulgierens unter 15° C gehalten wird.

Die Temperatur wird anschließend zur Verdampfung des organischen Lösungsmittels langsam erhöht. Anschließend wird die entstandene Suspension gefriergetrocknet.

Beispiel 2:

300 mg $\alpha$-Cyanacrylsäurebutylester werden in 1 ml Furan gelöst und diese Lösung in 10 ml 0,1 N Salzsäure, die 1 % Polyoxyethylenpolyoxypropylen-Polymer mit Molekulargewicht 12.000 (Pluronic® F127) enthält, emulgiert, wobei die Temperatur während des Emulgierens unter 15° C gehalten wird. Nach Abschluß der Polymerisation wird die entstandene Suspension gefriergetrocknet.

Beispiel 3:

200 mg $\alpha$-Cyanacrylsäurebutylester werden in 0,4 ml Isopren gelöst und in 30 ml 0,01 N Salzsäure, die 1 % Polyoxyethylenpolyoxypropylen-Polymer mit Molekulargewicht 8.350 (Pluronic® F68) enthält, emulgiert, wobei die Temperatur während des Emulgierens unter 10° C gehalten wird. Nach Abschluß der Polymerisation wird die Suspension mit 0,1 N NaOH neutralisiert und mit Natriumchlorid isotonisiert.

Beispiel 4:

400 mg $\alpha$-Cyanacrylsäurebutylester werden in 0,4 ml Methylenchlorid gelöst und in 60 ml 0,01 N Salzsäure, die 1 % Polyoxyethylenpolyoxypropylen-Polymer mit Molekulargewicht 12.000 (Pluronic® F127) enthält, emulgiert, wobei die Temperatur während des Emulgierens unter 10° C gehalten wird. Nach Abschluß der Polymerisation wird die Suspension mit 0,1 N Natronlauge neutralisiert und mit Natriumchlorid isotonisiert.

Beispiel 5:

400 mg Polycaprolacton werden in 6 ml Furan und 0,3 ml Cyclohexan gelöst und in 60 ml 1% Polyoxyethylenpolyoxypropylen-Polymer mit Molekulargewicht 12.000 (Pluronic® F127) emulgiert, wobei die Temperatur unter 15° C gehalten wird. Die Temperatur wird anschließend zur Verdampfung des organischen Lösungsmittels langsam erhöht. Danach wird die entstandene Suspension gefriergetrocknet.

Beispiel 6:

400 mg Terephthalsäuredichlorid werden in 2 ml Furan gelöst und in 50 ml 3%iger Natriumcarbonatlösung, die 0,1 % Polyoxyethylenpolyoxypropylen-Polymer mit Molekulargewicht 12.000 (Pluronic® F127) enthält, emulgiert Nach Zusatz von 60 mg L-Lysin, in 5 ml 0,1 %iger Pluronic F127 gelöst, werden die Mikrokapseln zentrifugiert und mehrmals mit 0,1 %iger Pluronic F127 Lösung ge waschen. Vor Gebrauch wird die Suspension mit Natriumchlorid isotonisiert.

Beispiel 7:

ß-Cyclodextrin-Isopentan-Einschlußverbindung:

100 ml gesättigte ß-Cyclodextrin-Lösung (1,8%ig) wird auf 10° C gekühlt und mit 3 ml Isopentan versetzt. Unter ständigem Rühren im Ultraschallbad wird der entstehende schwerlösliche Komplex ausgefällt. Durch Gefriertrocknen und Filtrieren wird der Niederschlag in kristalliner Form erhalten. Isopentangehalt nach GC-Bestimmung: 0,26 %

Beispiel 8:

ß-Cyclodextrin-2-Methyl-2-buten-Einschlußverbindung:

100 ml gesättigte ß-Cyclodextrin-Lösung (1,8%ig) wird auf 10° C gekühlt und mit 3 ml 2-Methyl-2-buten versetzt. Unter ständigem Rühren im Ultraschallbad wird der entstehende schwerlösliche Komplex ausgefällt. Durch Gefriertrocknen und Filtrieren wird der Niederschlag in kristalliner Form erhalten.

Beispiel 9:

ß-Cyclodextrin-2-Methyl-1-buten-Einschlußverbindung:

100 ml gesättigte ß Cyclodextrin-Lösung (1,8%ig) wird auf 10° C gekühlt und mit 3 ml 2-Methyl-1-buten ver setzt. Unter ständigem Rühren im Ultraschallbad wird der entstehende schwerlösliche Komplex ausgefällt. Durch Gefriergetrocknen und Filtrieren wird der Niederschlag in kristalliner Form erhalten. 2-Methyl-1-buten-Gehalt nach GC-Bestimmung: 0,82 %.

Beispiel 10:

ß-Cyclodextrin-Isopren-Einschlußverbindung:

100 ml gesättigte ß-Cyclodextrin-Lösung (1,8%ig) wird auf 10° C gekühlt und mit 3 ml Isopren versetzt. Unter ständigem Rühren im Ultraschallbad wird der entstehende schwerlösliche Komplex ausgefällt. Durch Gefriergetrocknen und Filtrieren wird der Niederschlag in kristalliner Form erhalten. Isoprengehalt nach GC-Bestimmung: 1,0%.

Beispiel 11:

ß-Cyclodextrin-Isopropylchlorid-Einschlußverbindung:

100 ml gesättigte ß Cyclodextrin-Lösung (1,8%ig) wird auf 10° C gekühlt und mit 3 ml Isopropylchlorid versetzt. Unter ständigem Rühren im Ultraschallbad wird der entstehende schwerlösliche Komplex ausgefällt. Durch Gefriertrocknen und Filtrieren wird der Niederschlag in kristalliner Form erhalten. Isopropylchloridgehalt nach GC-Bestimmung: 0,5 %.

Beispiel 12:

ß-Cyclodextrin-Isopentan-Einschlußverbindung:

100 ml gesättigte ß-Cyclodextrin-Lösung (1,8%ig) wird auf 10° C gekühlt und mit 3 ml Isopentan versetzt. Unter ständigem Rühren im Ultraschallbad wird der entstehende schwerlösliche Komplex ausgefällt. Durch Gefriertrocknen und Filtrieren wird der Niederschlag in kristalliner Form erhalten.

## Beispiel 13:

Xenon/α-Cyclodextrin-Einschlußverbindungen:

In einem 200 cm3 Autoklaven werden 100 ml gesättigte α-Cyclodextrin-Lösung (ca. 12%ig) unter 7 Atmosphären Xenon 7 Tage lang bei Zimmertemperatur inkubiert. Das kristalline Addukt kann abgesaugt, mit kaltem Wasser gewaschen und im Exsikkator über Calciumchlorid getrocknet werden.

## Beispiel 14:

Kohlendioxid/α-Cyclodextrin-Einschlußverbindung:

In einem 200cm3 Autoklaven werden 100 ml gesättigte α-Cyclodextrin-Lösung (ca. 12 %ig) unter 7 Atmosphären Kohlendioxid 7 Tage lang bei Zimmertemperatur inkubiert. Das kristalline Addukt kann abgesaugt, mit kaltem Wasser gewaschen und im Exsikkator über Calciumchlorid getrocknet werden.

## Beispiel 15:

Isopentan/Hydroxypropyl-ß-Cyclodextrin-Einschlußverbindung:

15 ml 20 %ige Hydroxypropyl-ß-Cyclodextrin-Lösung wurden bei 10°C mit 2 ml Isopentan versetzt, im Ultraschallbad 3 min beschallt und danach für 26 Stunden inkubiert. Der entstandene Komplex bleibt in Lösung.

## Beispiel 16:

Isopren/Hydroxypropyl-ß-Cyclodextrin-Einschlußverbindung:

15 ml 20 %ige Hydroxypropyl-ß-Cyclodextrin-Lösung werden bei 10°C mit 2 ml Isopren versetzt, im Ultraschallbad 3 min beschallt und danach für 26 Stunden inkubiert. Der entstandene Komplex bleibt zum Teil in Lösung und fällt zu einem Teil als weißer Niederschlag aus.

## Beispiel 17:

Furan/Hydroxypropyl-ß-Cyclodextrin-Einschlußverbindung:

15 ml 20 %ige Hydroxypropyl-ß-Cyclodextrin-Lösung werden bei 10°C mit 2 ml Furan versetzt, im Ultraschallbad 3 min beschallt und danach für 26 Stunden inkubiert. Der entstandene Komplex bleibt zum Teil in Lösung und fällt zu einem Teil als weißer Niederschlag aus.

## Beispiel 18:

Isopentan/α-Cyclodextrin-Einschlußverbindung:

20 ml gesättigte α-Cyclodextrin-Lösung werden mit 1 ml Isopentan versetzt und im Ultraschallbad 3 min beschallt. Der entstehende schwerlösliche Komplex wird durch Filtration gewonnen und über Calciumchlorid getrocknet.

## Beispiel 19:

Isopren/α-Cyclodextrin-Einschlußverbindung:

20 ml gesättigte α-CD-Lösung werden mit 1 ml Isopren versetzt und im Ultraschallbad 3 min beschallt. Der entstehende schwerlösliche Komplex wird durch Filtration gewonnen und über Calciumchlorid getrocknet.

## Beispiel 20:

Furan/α-Cyclodextrin-Einschlußverbindung:

20 ml gesättigte α-Cyclodextrin-Lösung werden mit 1 ml Furan versetzt und im Ultraschallbad 3 min beschallt. Der entstehende schwerlösliche Kommplex wird durch Filtration gewonnen und über Calciumchlorid getrocknet.

## Beispiel 21:

In einem Inkubationsgefäß werden zu 100 ml ges. α-Cyclodextrin-Lösung (5°C) unter Beschallung im Ultraschallbad 4 g Eukalyptol zugetropft und für weitere 30 min beschallt. Danach wird der Ansatz 48 Stunden in einem gekühlten, geschlossenen Gefäß geschüttelt. Der entstandene Niederschlag wird durch Filtration gewonnen, mit kaltem Ethanol gewaschen, in fl. $N_2$ eingefroren und gefriergetrocknet.

## Beispiel 22:

100 ml ges. ß-Cyclodextrin-Lösung werden mit 2 g Geraniol bei 5°C 4 h im Ultraschallbad beschallt und danach für 24 h bei 5°C inkubiert. Der entstandene Niederschlag wird abfiltriert, mit kaltem Ethanol gewaschen, in fl. $N_2$ eingefroren und gefriergetrocknet.

Für die Beispiele 17 - 22 gilt: Der kristalline Niederschlag wird nach dem Reinigen in einem geeigneten wäßrigen Medium, vorzugsweise physiologischer Kochsalz-, Glukose- oder Ringerlösung aufgenommen und ist dann injektionsfähig.

## Patentansprüche

1. Ultraschallkontrastmittel bestehend aus Mikropartikeln
dadurch gekennzeichnet, daß
die Mikropartikel aus Amylosen und einem Gas und/oder einer organischen Flüssigkeit mit einem Siedepunkt unter 60° C oder aus synthetischen bioabbaubaren Polymeren und einem Gas und/oder einer Flüssigkeit mit einem Siedepunkt unter 60° C bestehen.

2. Ultraschallkontrastmittel nach Anspruch 1, dadurch gekennzeichnet, daß
die Mikropartikel als Amylosen Cyclodextrine oder Cyclodextrinderivate enthalten.

3. Ultraschallkontrastmittel nach Anspruch 1, dadurch gekennzeichnet, daß
die Mikropartikel als synthetische bioabbaubare Polymere Polyester von α, ß, γ oder ε-Hydroxycarbonsäuren, Polyalkylcyanoacrylate, Polyaminosäuren, Polyamide, polyacrylierte Saccharide oder Polyorthoester enthalten.

4. Ultraschallkontrastmittel nach mindestens einem der Ansprüche 1 - 3,

dadurch gekennzeichnet, daß
die Mikropartikel als organische Flüssigkeiten
mit einem Siedepunkt unter 60° C
1.1-Dichlorethylen,
2-Methyl-2-buten,
Isopropylchlorid,
2-Methyl-1.3-butadien,
2-Butin,
2-Methyl-1-buten,
Dibromdifluormethan,
Furan,
3-Methyl-1-buten,
Isopentan,
Diethylether,
3.3-Dimethyl-1-butin,
Dimethylaminoaceton,
Propylenoxid,
N-Ethylmethylamin,
Brommethan,
N-Ethyldimethylamin,
Methylenchlorid,
Pentan,
Cyclopentan,
2.3.-Pentadien,
Cyclopenten
oder deren Gemische enthalten.

5. Ultraschallkontrastmittel nach mindestens einem der Ansprüche 1 - 3,
dadurch gekennzeichnet, daß,
die Mikropartikel als Gase
Luft,
Edelgase,
Stickstoff,
Sauerstoff,
Kohlendioxid,
Wasserstoff,
Ammoniak,
Ethylen,
Methan,
Ethan,
Propan oder
Butan
oder deren Gemische enthalten.

6. Ultraschallkontrastmittel nach 1,
dadurch gekennzeichnet, daß
die Mikropartikel ätherische Öle enthalten.

7. Ultraschallkontrastmittel nach mindestens einem der Ansprüche 1,2 und 4 - 6,
dadurch gekennzeichnet, daß
die aus Amylosen bestehenden Mikropartikel mit einer hydrophoben Hüllsubstanz bestehend aus Ölen, Fetten und/oder oberflächenaktiven Substanzen überzogen und in wäßrigem Medium suspendiert sind.

8. Ultraschallkontrastmittel nach mindestens einem der Ansprüche 1, 2 und 4 - 7,
dadurch gekennzeichnet, daß
die aus Amylosen bestehenden Mikropartikel von einer Matrix, insbesondere polymerer Struktur, umhüllt sind.

9. Ultraschallkontrastmittel nach mindestens einem der Ansprüche 1 - 8,
dadurch gekennzeichnet, daß
durch Zugabe osmotisch aktiver Substanzen, insbesondere Kochsalz, Mannit, Galaktose, Glukose, Fruktose, die physiologische Isotonie eingestellt ist.

10. Verfahren zur Herstellung von Ultraschallkontrast mitteln mit Mikropartikeln aus synthetischen bioabbaubaren Polymeren nach einem der Ansprüche 1 oder 3 - 5,
dadurch gekennzeichnet, daß
ein Polymer oder Copolymer in einem oder mehreren, mit Wasser nicht mischbaren, organischen Lösungsmitteln gelöst und anschließend ggf. nach Zusatz eines weiteren Lösungsmittels in Wasser emulgiert wird und die erhaltene Emulsion anschließend filtriert, ggf. getrocknet wird.

11. Verfahren zur Herstellung von Ultraschallkontrastmitteln mit Mikropartikeln aus synthetischen bioabbaubaren Polymeren nach einem der Ansprüche 1 oder 3 - 5,
dadurch gekennzeichnet, daß
ein Polymer oder Copolymer in einem oder mehreren, Gasblasen enthaltenden Lösungsmittel gelöst und anschließend ggf. nach Zusatz eines weiteren Lösungsmittels oder eines weiteren Polymeren ausgefällt oder in Wasser emulgiert und die erhaltene Suspension oder Emulsion anschließend filtriert, ggf. getrocknet wird.

12. Verfahren nach Anspruch 10 oder 11,
dadurch gekennzeichnet, daß
als Lösungsmittel Furan, Pentan, Aceton, Dioxan, Ethylacetat, p-Xylol, Methylenchlorid, Cyclohexan oder n-Hexan oder ein daraus bestehendes Lösungsmittelgemisch verwendet wird.

13. Verfahren nach Anspruch 10 oder 11,
dadurch gekennzeichnet, daß
der Emulsion ein Emulgator zugesetzt wird.

14. Verfahren zur Herstellung von Ultraschallkontrastmitteln mit Mikropartikeln aus synthetischen, bioabbaubaren Polymeren nach einem der Ansprüche 1 und/oder 3 -6,
dadurch gekennzeichnet, daß
ein Monomer in einem oder mehreren organischen Lösungsmitteln gelöst und in 5 - 30 Teilen Wasser oder 0,01 - 0,1 N Salzsäure ggf. unter Zusatz von Emulgatoren oder Puffersubstanzen bei einer Temperatur unterhalb des Siedepunkts des organischen Lösungsmittels emulgiert wird und dieser Emulsion eine 0,2% - 20%ige wäßrige Lösung eines zweiten Monomeren oder ggf. die Lösung einer pH-Wert erhöhenden Substanz zugegeben und ggf. getrocknet wird.

15. Verfahren zur Herstellung von Ultraschallkontrastmitteln mit Mikropartikeln aus synthetischen, bioab baubaren Polymeren nach einem der Ansprüche 1 und/oder 3 - 6,
dadurch gekennzeichnet, daß
ein Monomer in einer oder mehreren, gasblasenenthaltenden Flüssigkeiten ggf. unter Zusatz von Emulgatoren und/oder Puffersubstanzen gelöst oder dispergiert wird und dieser Lösung oder Dispersion ggf. eine 0,2% - 20%ige Lösung eines zweiten Monomeren oder eine pH-Wert erhöhende Substanz in gelöster oder gasförmiger Form zugegeben und ggf. getrocknet werden.

16. Verfahren nach Anspruch 14 oder 15, dadurch gekennzeichnet, daß als erstes Monomer Terephthaloyl- oder Sebacoylchlorid oder Cyanacrylsäureester, als zweites Monomer L-Lysin und als organisches Lösungsmittel 2-Methyl-1.3-butadien, Methylenchlorid, Toluol, Dioxan oder Cyclohexan verwendet wird.

17. Verfahren zur Herstellung von Ultraschallkontrastmittel mit Mikropartikeln aus synthetischen bioabbaubaren Polymeren nach einem der Ansprüche 1 und/oder 3 - 6, dadurch gekennzeichnet, daß in einer 0,5 - 10%igen wäßrigen Lösung eines Monomeren, die ggf. Zusätze wie Emulgatoren (0,01 - 5 %) oder Quasiemulgatoren (0,1 - 5 %) enthält, Gasblasen erzeugt und danach eine quervernetzende Substanz und/oder ein Reaktionsstarter zugesetzt werden.

18. Verwendung der Ultraschallkontrastmittel nach einem Ansprüche 1 - 9 für diagnostische oder therapeutische Verfahren.

Europäisches
Patentamt

**EUROPÄISCHER RECHERCHENBERICHT**

Nummer der Anmeldung

EP 89 73 0021

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 123 235 (SCHERING AG)<br>* Insgesamt, insbesondere Seite 6, Zeilen 19-21; Seite 7, Zeilen 7-11 *<br>--- | 1,2,5-9,18 | A 61 K 49/00 |
| A | WO-A-8 002 365 (RASOR ASSOCIATES, INC.)<br>* Ansprüche *<br>--- | 1-18 | |
| A | FR-A-2 429 616 (DOW CORNING CORP.)<br>* Seite 18; Beispiel 4 *<br>--- | 1 | |
| A | FR-A-2 496 460 (CHINOIN)<br>* Seiten 6-8; Beispiele 1-5 *<br>--- | 1,6 | |
| A | DE-A-3 341 001 (KRAUSE et al.)<br>* Insgesamt *<br>----- | 1,3,10-17 | |
| | | | **RECHERCHIERTE SACHGEBIETE (Int. Cl.4)**<br><br>A 61 K<br>A 61 B |

Der vorliegende Recherchenbericht wurde für alle Patentansprüche erstellt

| Recherchenort | Abschlußdatum der Recherche | Prüfer |
|---|---|---|
| DEN HAAG | 12-05-1989 | BENZ K.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

EPO FORM 1503 03.82 (P0403)